(19) Europäisches Patentamt
European Patent Office
Office européen des brevets

(11) Publication number: 0 142 283 B1

(12) EUROPEAN PATENT SPECIFICATION

(45) Date of publication of patent specification: 30.01.91

(21) Application number: 84307102.8

(22) Date of filing: 17.10.84

(60) Divisional application 90200019.9 filed on 17/10/84.

(51) Int. Cl.[5]: C 07 C 215/30, C 07 C 215/22, C 07 C 323/01, C 07 C 275/28, C 07 C 233/64, C 07 C 237/02, C 07 C 317/02, C 07 C 215/46 // C07C43/23, C07C43/174, C07C255/00, C07D263/14, C07D317/28, C07C65/03, C07C69/157, C07C69/612, C07C205/20, C07C47/575

# (54) Phenylethylamines, process for their preparation and compositions containing them.

(30) Priority: 25.10.83 GB 8328489
25.10.83 GB 8328490
06.12.83 GB 8332447
06.12.83 GB 8332448
06.12.83 GB 8332452
24.01.84 GB 8401746
24.01.84 GB 8401747
24.01.84 GB 8401748
24.01.84 GB 8401750

(43) Date of publication of application: 22.05.85 Bulletin 85/21

(45) Publication of the grant of the patent: 30.01.91 Bulletin 91/05

(84) Designated Contracting States: AT BE CH DE FR GB IT LI LU NL SE

(56) References cited:
EP-A-0 072 061
CH-A- 377 797
FR-A-2 140 149
US-A-2 653 977
US-A-3 960 959
US-A-4 181 738

The file contains technical information submitted after the application was filed and not included in this specification

(73) Proprietor: FISONS plc
Fison House Princes Street
Ipswich Suffolk IP1 1QH (GB)

(72) Inventor: Dixon, John
Church Farmhouse Great Dalby
Nr Melton Mowbray Leicestershire (GB)
Inventor: Ince, Francis
78 Leconfield Road
Loughborough Leicestershire (GB)
Inventor: Tinker, Alan Charles
97 Knightthorpe Road
Loughborough Leicestershire (GB)

(74) Representative: Craig, Christopher Bradberry et al
Fisons plc 12 Derby Road
Loughborough Leicestershire LE11 0BB (GB)

EP 0 142 283 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to new compounds, processes for their preparation and compositions containing them.

European Patent No. 72061 discloses a number of phenylethylamine derivatives which exhibit pharmacological activity in animals and are indicated for use in the treatment of *inter alia* congestive heart failure. We have now found a new group of phenylethylamine derivatives with advantageous properties.

Thus, according to the invention we provide the compounds of formula I,

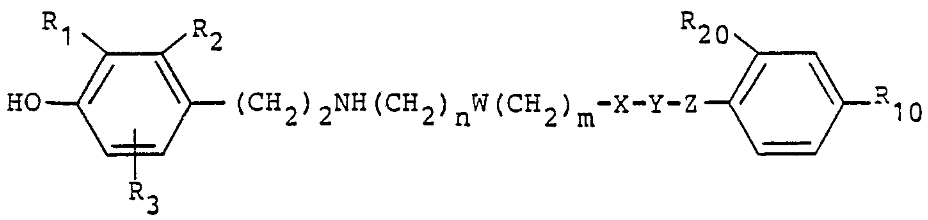

I

in which

$R_1$ represents OH,

$R_2$ and $R_3$, which may be the same or different,
each independently represent hydrogen, fluorine, chlorine, bromine, alkyl C1 to 6, nitro, $(CH_2)_pR_9$, or $SR_9$,

W represents a single bond,

X represents NH,

Y represents $(CH_2)_q$ and $R_{20}$ represents hydrogen,

Z represents a single bond,

n and m each independently represent an integer from 1 to 4 inclusive,

q represents an integer from 1 to 3 inclusive,

p represents 0 or an integer from 1 to 3 inclusive,

$R_9$ represents phenyl or phenyl substituted by hydroxy, and

$R_{10}$ represents hydrogen or chlorine,

provided that $R_2$ and $R_3$ do not both represent hydrogen,
and pharmaceutically acceptable salts thereof.

The invention also provides the compounds of formula I, and their pharmaceutically acceptable salts, as pharmaceuticals.

According to our invention we also provide a process for the production of a compound of formula I, or a pharmaceutically acceptable salt thereof, which comprises deprotecting a compound of formula II,

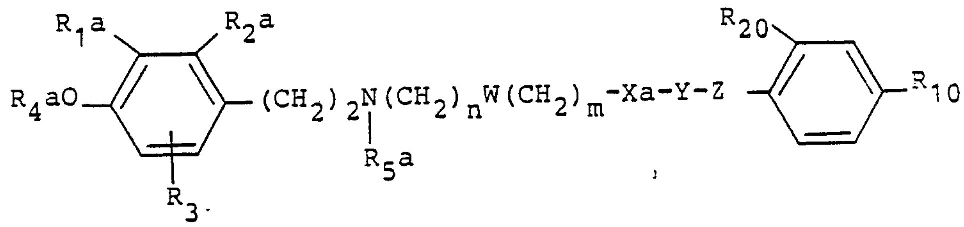

II

in which

$R_3$, $R_{10}$, $R_{20}$, n, m, W, Y and Z are as defined above,

$R_4a$ and $R_5a$, which may be the same or different, each represent hydrogen or a protecting group,

$R_1a$, $R_2a$ and Xa have the same respective meanings as $R_1$, $R_2$ and X defined above, save that in addition

$R_1a$ represents $OR_6a$ in which $OR_6$ may represent a protecting group,

Xa may represent $NR_8a$, in which $R_8a$ represents a protecting group,

provided that the compound of formula II bears at least one protecting group,

and where desired or necessary converting the resulting compound of formula I to a pharmaceutically acceptable salt thereof, or vice versa.

Protecting groups that $R_4a$, $R_5a$, $R_6a$ and $R_8a$ may represent include, for example, alkyl C1 to 6, especially methyl; phenylalkyl C7 to 12, especially benzyl; alkanoyl C2 to 6, such as acetyl; and haloalkanoyl C2 to 6, especially trifluoroacetyl. In addition, the protecting group may protect two functional groups, for example, $R_4a$ and $R_6a$ may together represent $(CH_3)_2C$. Other protecting groups are well known and include those described in Protective Groups in Organic Chemistry, Ed: J. W. F. McOmie, Plenum Press (1973), and Protective Groups in Organic Synthesis, T. W. Greene, Wiley-Interscience (1981).

Removal of the protecting group depends on the nature of the protecting group; conventional techniques may generally be employed, including acidic or basic cleavage or hydrogenolysis. For example,

protecting alkyl or phenylalkyl groups may be removed by cleavage using a protic acid e.g. hydrochloric acid or hydrobromic acid at a temperature of from 0 to 150°C, or a Lewis acid, e.g. by reacting with boron trihalide in a hydrocarbon solvent. When the protecting group is alkanoyl or haloalkanoyl, cleavage may be effected using a base, e.g. sodium hydroxide, in a suitable solvent, e.g. aqueous ethanol. Lewis bases, e.g. pyridine hydrochloride, may be used to cleave alkyl or phenylalkyl groups. 1-Phenylalkyl groups, e.g. benzyl, may be removed by catalytic hydrogenation using a suitable catalyst, e.g. palladium, in a suitable solvent, e.g. ethanol, or acetic acid. Further methods for the removal of protecting groups are described in both McOmie and Greene, loc cit. Both McOmie and Greene also described numerous methods for the application of protective groups.

Compounds of formula II may be made by reducing a compound of formula III,

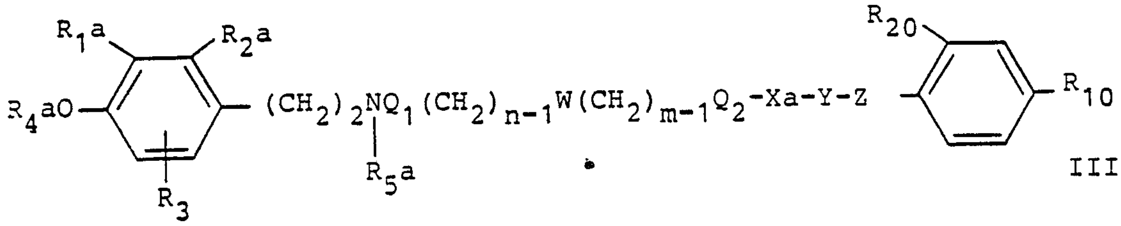

III

in which one or both of $Q_1$ and $Q_2$ represents CO, and the other represents $CH_2$, and $R_1a$, $R_2a$, $R_3$, $R_4a$, $R_5a$, $R_{10}$, $R_{20}$, W, Xa, Y, Z, n and m are as defined above.

The reducing agent may be electrophilic, for example diborane, or nucleophilic, for example, a complex metal hydride such as lithium aluminium hydride or sodium (2-methoxyethoxy)aluminium hydride. The reaction may be carried out in a suitable solvent inert to the reaction conditions. Aprotic solvents are preferred, for example tetrahydrofuran, diethyl ether or 1,2-dimethoxyethane. The reaction may be carried out at a temperature of, for example, from 0 to 100°C.

When Xa represents $NR_8a$, the compounds of formula III may be made by sequentially reacting the groups $L_1$, and $L_2$, in any order of the corresponding compound of formula IV,

$$L_1Q_1—(CH_2)_{n-1}W(CH_2)_{m-1}Q_2L_2 \qquad IV$$

in which one of $L_1$ and $L_2$ represents a good leaving group and the other of $L_1$ and $L_2$ represents either a good leaving group or a group which may be readily converted into a good leaving group, and W, n, m, $Q_1$ and $Q_2$ are as defined above, with the compounds of formula V and formula VI,

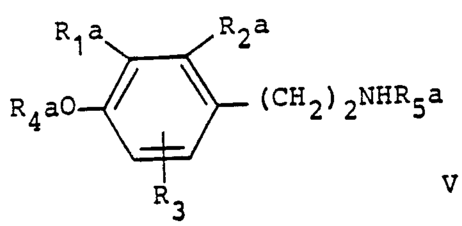

V

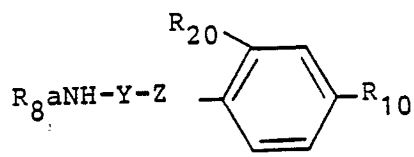

VI

in any order, wherein $R_1a$, $R_2a$, $R_3a$, $R_4a$, $R_5a$, $R_8a$, $R_{10}$, $R_{20}$, Y, and Z are as defined above.

Good leaving groups that $L_1$ and $L_2$ may represent include, for example, halogen, e.g. chlorine or bromine; 1-imidazolyl, trifluoromethanesulphonate; alkyl carbonate, e.g. ethyl carbonate, benzyl carbonate; alkanoyloxy, e.g. acetoxy, or trifluorocetoxy.

The displacement reactions may be carried out in a solvent which is inert to the reaction conditions, for example, a chlorinated hydrocarbon, e.g. chloroform, in the presence of a non-nucleophilic base, e.g. triethylamine. The reaction may be carried out at a temperature of from about 0 to 100°C.

The free acids of compound IV, i.e. those compounds in which both $L_1$ represents —OH and $Q_1$ represents CO, and/or both $L_2$ represents —OH and $Q_2$ represents CO may be reacted, e.g. with thionyl chloride, ethyl chloroformate, or N,N'-carbonyldiimidazole to convert the carboxyl groups to a group $—COL_1$ or $—COL_2$ respectively.

When $L_1$ and/or $L_2$ represent a group which may be converted into a good leaving group, such convertable groups include alkoxy, e.g. ethoxy or methoxy; and hydroxy. The conversion may be effected using conventional techniques.

An example of a sequential replacement of $L_1$ and $L_2$ is as follows:

A compound of formula IV in which $L_1$ represents $OCH_3$, $L_2$ represents OH and both $Q_1$ and $Q_2$ represent

CO is reacted with a compound of formula VI in dichloromethane, at 0°C with N,N'-carbonyldiimidazole, to give the compound of formula VII,

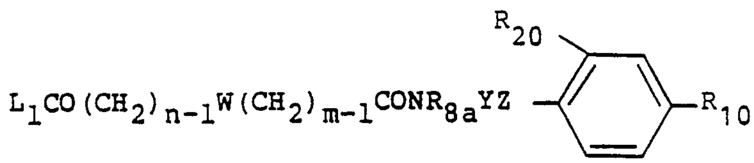

VII

in which $L_1$ represents $OCH_3$, and $R_8a$, $R_{10}$, $R_{20}$, n, m, W, Y, and Z are as defined above.

Saponification of the $-COL_1$ group with one equivalent of base, followed by acidification gives the corresponding compound of formula VII with $L_1$ representing $-OH$, which can be reacted with the appropriate compound of formula V in the presence of N,N'-carbonyldiimidazole to give the desired compound of formula III.

Using analogous processes, the following compounds may be produced:

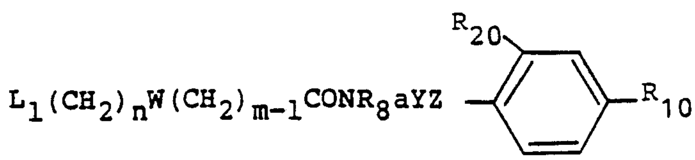

VIII

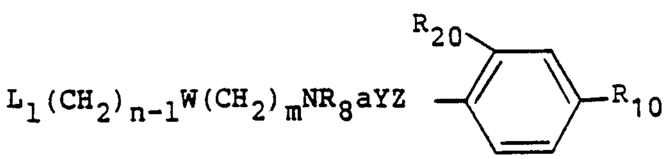

IX

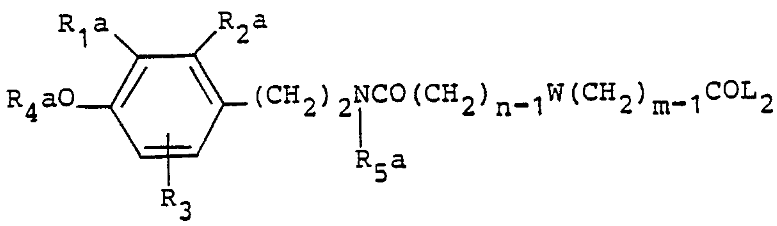

X

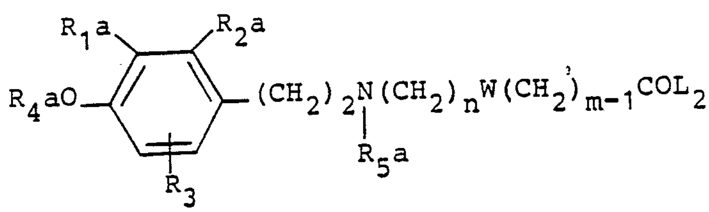

XI

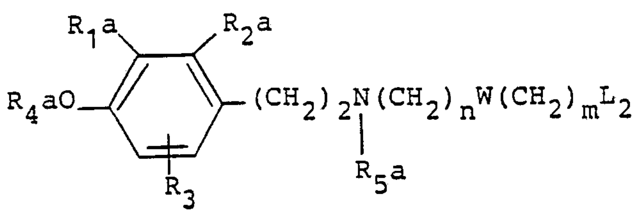

XII

in which $R_1a$, $R_2a$, $R_3$, $R_4a$, $R_5a$, $R_8a$, $R_{10}$, $R_{20}$, $L_1$, $L_2$, n, m, W, Y, and Z are as defined above.

Compounds of formula II may also be made by reacting a corresponding compound of formula XIV,

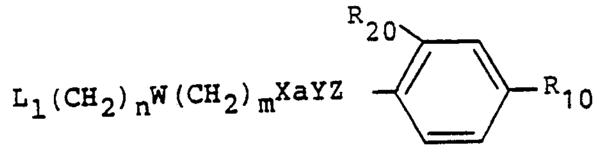

XIV

in which $R_{10}$, $R_{20}$, n, m, W, Xa, Y, Z and $L_1$ are as defined above, with a compound of formula V as defined above.

The reaction is preferably carried out in the presence of a base. As a specific example, $L_1$ may represent bromine, $R_5a$ may represent trifluoroacetyl, the reaction being carried out in dimethylformamide in the presence of sodium hydride.

The compounds of formulae IV, V and VI are either known or may be made from known compounds by conventional techniques, known per se.

The acid addition salts of the compounds of formula I may be prepared by reacting of the free base with an appropriate acid. The acid addition salts may be converted to the corresponding free base by the action of a stronger base.

The processes as described above may produce the compound of formula I or a salt thereof. It is also within the scope of this invention to treat any salt so produced to liberate the free compound of formula I, or to convert one salt into another.

The compounds of formula I and the intermediates therefor may be isolated from their reaction mixtures using conventional techniques.

Pharmaceutically acceptable salts of the compounds of formula I include pharmaceutically acceptable acid addition salts. Suitable salts include salts of mineral acids, for example, hydrohalic acids, e.g. hydrochloric acid or hydrobromic acid; or organic acids, e.g. formic, acetic or lactic acids. The acid may be polybasic, for example sulphuric, fumaric or citric acid.

Other pharmaceutically acceptable derivatives are compounds which will be suitable bioprecursors (prodrugs) of the compounds of formula I and will be readily apparent to those skilled in the art and may be made from the compounds of formula I using conventional processes known *per se* or by processes analogous to those described above. Suitable bioprecursors include amides, e.g. acetamides or benzamides, of compounds of formula I, and esters, e.g. alkanoyl, such as acetyl or isobutyryl, or aroyl C7—9 e.g. benzoyl, esters.

$R_2$ or $R_3$, when they represent alkyl C1 to 6 preferably contain up to and including four carbon atoms. Specific groups that $R_2$ and $R_3$ may represent include methyl, ethyl, n-propyl, isopropyl and tert-butyl.

We prefer $R_2$ and $R_3$ to be selected from hydrogen, fluorine, chlorine, bromine, $CH_2CH_2C_6H_5$ and $CH_2CH_2C_6H_4OH$. We particularly prefer compounds in which $R_1$ is OH, $R_2$ represents chlorine and $R_3$ represents hydrogen. We also particularly prefer compounds in which $R_1$ is OH and $R_3$ represents fluoro.

n and m each independently preferably represent 1, 2 or 3.

q preferably represents 1 or 2.

p preferably represents 0, 1 or 2.

We prefer the sum of n + m to be from 5 to 7 inclusive, especially 6.

The compounds of formula I, and pharmaceutically acceptable salts thereof, are useful because they possess pharmacological activity in animals. Thus the compounds act on peripheral and/or central dopamine receptors. As such, they lower blood pressure, reduce heart rate and increase blood flow to certain vascular beds, e.g. renal beds. Some compounds also have an action on other adrenoreceptors, and these exhibit cardiac stimulant and bronchodilator effects. Activity of the compounds has been observed in the following assay systems:

(a) canine renal blood flow, McNay and Goldberg, J. Pharmac, Exp. Ther., 151, 23—31, 1966.

(b) rabbit isolated ear artery, McCullough, Rand and Story, Br. J. Pharmac, 49, 141—142, 1973, and

(c) cat nictitating membrane, Gyorgy and Doda, Arch. Int. Pharmacodyn, 226, 194—206, 1977.

The compounds of the invention are indicated for use in the treatment of congestive heart failure, renal failure, angina pectoris, ischaemis heart disease, hypertension and reversible obstructive airways disease, hyperprolactinaemia and also in Parkinson's disease and other neurological disorders. Compounds of the invention are also indicated for use in the treatment of glaucoma, gastric hypersecretion, e.g. in peptic ulcers, premature labour, acromegaly, and improvement of the blood supply to and healing of intestinal anastomoses and stomata.

The dosage administered will naturally depend on the compound employed, the mode of administration and the desired effect. However, in general, satisfactory results are obtained when the compounds are administered at a dosage of from 0.05 µg to 50 mg per kilogram of body weight per day. For man, the indicated total daily dosage is in the range 2.5 µg to 3.5 g, which may be administered in divided doses of, for example 1 µg to 750 mg.

The new compounds of the present invention may be used in combination with, or sequentially with, a wide variety of other pharmaceutically active substances. Where appropriate the compounds may be mixed with one or more oher active substances. The particular mixture or dose regimen used, and ratio of

the active ingredients will depend on a variety of factors including the condition to be treated, the mode of administration, the particular active ingredients and the patient concerned.

Examples of compounds with which the present compounds may be mixed include:

beta-blockers, especially cardioselective beta blockers, for example, atenolol;

diuretics, for example thiazides, e.g. furosemide;

angiotensin converting enzyme inhibitors, for example captopril;

inotropic agents, for example, amrinone;

antimetics, for example, sulpiride, metoclopramide, or domperidone.

The compounds of formula I, and pharmaceutically acceptable salts thereof, have the advantage that they are more efficacious or produce less undesirable side effects in certain pharmacological models, or are longer acting than compounds of similar structure to the compounds of formula I.

The compounds of the invention may be administered by a wide variety of routes and may act systemically or locally. Thus the compounds may be administered by oral or nasal inhalation to the lung, to the buccal cavity, oesophageally, rectally, topically to the skin or to other available surfaces of the body, e.g. the eye, by injection, e.g. intravenously, intramuscularly, intraperitoneally, by instillation or by surgical implant.

According to our invention we also provide a pharmaceutical composition comprising preferably less than 80%, and more preferably less than 50%, by weight of a compound of formula I, or a pharmaceutically acceptable salt thereof, in admixture with a pharmaceutically acceptable adjuvant, diluent or carrier. Examples of suitable adjuvants, diluents or carriers are: for tablets, capsules and dragees; microcrystalline cellulose, calcium phosphate, diatomaceous earth, a sugar such as lactose, dextrose or mannitol, talc, stearic acid, starch, sodium bicarbonate and/or gelatin;

for suppositories; natural or hardened oil or waxes; and for inhalation compositions, coarse lactose.

When the compounds are to be used in aqueous solution it may be necessary to incorporate a chelating or sequestering agent, e.g. sodium edetate, an antioxidant, e.g. sodium metabisulphite or buffering agents, e.g. sodium hydrogen phosphate and sodium phosphate. Aqueous solutions typically contain up to about 10% w/w of the new compound and may be used for intravenous injections.

According to the invention, we further provide a method of increasing the force of contraction of the heart in an animal, either human or non-human, which method comprises administering to the animal an effective amount of one or more compounds of the invention.

The invention is illustrated, but in no way limited by the following Examples in which temperatures are in degrees Centigrade.

In general all compounds all compounds and intermediates are named in accord with "Naming and Indexing of Chemical substances for Chemical Abstracts", reprinted from Appendix IV of the Chemical Abstracts 1977 Index Guide.

In particular derivatives of hexanedioic acid, in which the carboxylic acid groups are in a 1,6-relation to one another, are named as hexanedioates, not 1,6-hexanedioates.

## Example 1

5-[2-(6-(2-Phenylethylamino)hexylamino)ethyl]-3-propyl-1,2-benzenediol

a) *3,4-Dimethoxy-5-propylbenzenemethanol*

A solution of 3,4-dimethoxy-5-propylbenzaldehyde (11 g) and sodium borohydride (2 g) in 2-propanol (100 ml) was stirred at room temperature for 2 hours. The mixture quenched with 2N HCl (200 ml) and extracted with ethyl acetate. The organic phase was separated, washed with brine, dried over $MgSO_4$, filtered and evaporated to leave a yellow oil which was purified by Kugelrohr bulb to bulb distillation at 1 mm Hg air bath temperature 150° to give the sub-title compound as a colourless oil (8 g).

The following compounds were prepared by the method of (a) above:

i) 3,4-Dimethoxy-2-propylbenzenemethanol; mp, 70—71.5°;

ii) 3-Fluoro-4,5-dimethoxybenzenemethanol; m/e 186;

iii) 2-Fluoro-3,4-dimethoxybenzenemethanol; m/e 186;

b) *3,4-Dimethoxy-5-propylbenzeneacetonitrile*

A solution of the alcohol from step (a) (7.89 g) and thionyl chloride (5.5 ml) in dry dichloromethane was heated under reflux for 2 hours. The solution was evaporated to dryness.

A solution of the crude chloride in dry dimethylsulphoxide (20 ml) was added to a suspension of sodium cyanide (3.68 g) in dry dimethylsulphoxide (20 ml). The mixture was stirred at 20° for 18 hours then quenched with water (100 ml) and extracted with ethyl acetate. The organic phase was washed with brine, dried over magnesium sulphate and evaporated on a steam bath until HCN had ceased to be evolved. Further evaporation gave an oil which was purified by Kugelrohr distillation at 1 mm Hg and air bath temperature 200° to give the sub-title compound as a pale yellow oil (7.3 g).

The following compounds were prepared by the method of 1 (b):

i) 3,4-Dimethoxy-2-propylbenzeneacetonitrile; mp, 75—76°;

ii) 3,4-Dimethoxy-5-nitrobenzeneacetonitrile; MS m/e 222;

iii) 3,4-Dimethoxy-2-[2-phenylethyl]benzeneacetonitrile; Bp 200° 1 mm Hg;

iv) 2-Ethyl-3,4-dimethoxybenzeneacetonitrile; Bp 170° 0.5 mm Hg;
v) 2-Butyl-3,4-dimethoxybenzeneacetonitrile; mp 43—44°;
vi) 3,4-Dimethoxy-2-[1-methylethyl]benzeneacetonitrile; Bp 200°/0.35 mm Hg;
vii) 5,6-Dimethoxy-[1,1'-biphenyl]-2-acetonitrile; Bp 200°/0.5 mm Hg;
viii) 3,4-Dimethoxy-2-phenylmethylbenzeneacetonitrile; Bp 200°/0.25 mg Hg;
ix) 3,4-Dimethoxy-2-phenylthiobenzeneacetonitrile; mp 79—80°;
x) 4',5,6-Trimethoxy-[1,1'-biphenyl]-2-acetonitrile; mp 105—106°;
xi) 2-[2-[4-Methoxyphenyl]ethyl]-3,4-dimethoxybenzeneacetonitrile; mp 91—93°;
xii) 5-Fluoro-3,4-dimethoxy-2-propylbenzeneacetonitrile; Bp 150°/0.2 mm Hg;
xiii) 3-Fluoro-4,5-dimethoxybenzeneacetonitrile; MS m/e 195;
xiv) 2-Fluoro-4,5-dimethoxybenzeneacetonitrile; mp 116.3—117.5°;

c) *3,4-Dimethoxy-5-propylbenzeneethanamine hydrochloride*

A solution of the nitrile from step (b) (8.76 g) in dry tetrahydrofuran (100 ml) was stirred under an atmosphere of nitrogen during the addition of a 1M solution of borane in tetrahydrofuran (120 ml). The solution was heated under reflux for 18 hours. Methanol (100 ml) was added to the cooled reaction mixture and the solution evaporated to dryness. The residue was dissolved in methanolic/HCl (100 ml) and heated under reflux for 4 hours. The solution was evaporated and the solid crystallised from ethyl acetate/cyclohexane as colourless prisms (5.3 g) mp 116—117°.

The following compounds were prepared by the method of (c):
i) 3,4-Dimethoxy-2-propylbenzeneethanamine hydrochloride; mp 221—223°;
ii) 3,4-Dimethoxy-2-nitrobenzeneethanamine hydrochloride; mp 179.8—181.8°;
iii) 3,4-Dimethoxy-5-nitrobenzeneethanamine hydrochloride; mp 179.3—181.3°;
iv) 3,4-Dimethoxy-2-(2-phenylethyl)benzeneethanamine hydrochloride; mp 201—203°;
v) 2-Ethyl-3,4-dimethoxybenzeneethanamine hydrochloride; mp 253—255°;
vi) 2-Butyl-3,4-dimethoxybenzeneethanamine hydrochloride; mp 136—137°;
vii) 3,4-Dimethoxy-2-[1-methylethyl]benzeneethanamine hydrochloride; mp 193.9—195.2°;
vii) 5,6-Dimethoxy-[1,1'-biphenyl]-2-ethanamine hydrochloride: mp 218—219°;
ix) 3,4-Dimethoxy-2-(phenylmethyl)benzeneethanamine hydrochloride; mp 143.3—144.6°;
x) 3,4-Dimethoxy-2-(phenylthio)benzeneethanamine hydrochloride; mp 106—108°;
xi) 4',5,6-Trimethoxy-[1,1'-biphenyl]-2-ethanamine hydrochloride; mp 177—178°;
xii) 2-[2-[4-Methoxyphenyl]ethyl]-3,4-dimethoxybenzene ethanamine hydrochloride; mp 135—137°;
xiii) 2-Fluoro-4,5-dimethoxybenzeneethanamine hydrochloride; mp 158—160°;
xiv) 3-Fluoro-4,5-dimethoxybenzeneethanamine hydrochloride; mp 161.7—163.0;
xv) 5-Fluoro-3,4-dimethoxy-2-propylbenzeneethanamine hydrochloride; mp 232.7—234.0°;

d) *N-[2-(3,4-Dimethoxy-5-propylphenyl)ethyl-N'-[2-phenylethyl]hexanediamide*

A solution of 6-oxo-6-(2-phenylethylamino)hexanoic acid (2.05 g) and N,N'-carbonyldiimidazole (1.33 g) in dry dichloromethane (50 ml) was stirred at 20° for 1.5 hours and a solution of 3,4-Dimethoxy-5-propylbenzenethanamine (1.8 g) in dichloromethane (20 ml) then added. The mixture was stirred at 20° for 18 hours and water (50 ml) added. The organic phase was separated, washed with dilute hydrochloric acid, aqueous sodium bicarbonate and brine, dried over $MgSO_4$ and evaporated to leave a colourless solid. Crystallisation from 2-propanol/cyclohexane gave the bis amide as colourless prisms (2.7 g) mp 128—129°.

The following compounds were prepared by the method of 1 (d):
i) N-[2-(3,4-Dimethoxy-2-propylphenyl)ethyl]-N'-[2-phenylethyl]hexanediamide; mp 137.8—138.5°;
ii) N-[2-(3,4-Dimethoxy-2-methylphenyl)ethyl]-N'-[2-phenylethyl]hexanediamide; mp 165—167°;
iii) N-[2-(3,4-Dimethoxy-2-nitrophenyl)ethyl]-N'-[2-phenylethyl]hexanediamide; mp 143.2—144.5°;
iv) N-[2-(3,4-Dimethoxy-5-nitrophenyl)ethyl]-N'-[2-phenylethyl]hexanediamide; mp 154.8—156°;
v) N-[2-(2-Chloro-3,4-dimethoxyphenyl)ethyl]-N'-[2-phenylethyl]hexanediamide; mp 162—165°;
vi) N-[2-(3,4-Dimethoxyphenyl)-2-methyl]propyl-N'-[2-phenylethyl]hexanediamide; mp 94—96°;
vii) N-[2-[3,4-Dimethoxy-2-[2-phenylethyl]phenyl]ethyl]-N'-[2-phenylethyl]hexanediamide; mp 158—160°;
viii) N-[2-[2-Ethyl-3,4-dimethoxyphenyl]ethyl]-N'-[2-phenylethyl]hexanediamide; mp 154—156°;
ix) N-[2-[2-Butyl-3,4-dimethoxyphenyl]ethyl]-N'-[2-phenylethyl]hexanediamide; mp 138—139°-;
x) N-[2-[3,4-Dimethoxy-2-[1-Methylethyl]phenyl]ethyl]-N'[2-phenylethyl]hexanediamide; mp 111.6—112.8°;
xi) N-[2-[[1,1'-Biphenyl]-2-yl-5,6-dimethoxy]ethyl]-N'-[2-phenylethyl]hexanediamide; mp 146—147°;
xii) N-[2-[2,3,4-Trimethoxyphenyl]ethyl]-N'-[2-phenylethyl]hexanediamide; mp 127—129°;
xiii) N-[2-[3,4-Dimethoxy-2-(phenylmethyl)phenyl]ethyl]-N'-[2-phenylethyl]hexanediamide; mp 137.8—138.6°;
xiv) N-[2-[3,4-Dimethoxy-2-(phenylthio)phenyl]ethyl]-N'-[2-phenylethyl]hexanediamide; mp 137.6—138.1°;
xv) N-[2-[[1,1'-Biphenyl]-2-yl-4',5,6-trimethoxy]ethyl]-N'-[2-phenylethyl]hexanediamide; mp 187—189°;

xvi) N-[2-[2-[2-[4-methoxyphenyl]ethyl]-3,4-dimethoxyphenyl]ethyl]-N'-[2-phenylethyl]hexanediamide; mp 152—154°;

xvii) N-[2-[2-Fluoro-3,4-dimethoxyphenyl]ethyl]-N'-[2-phenylethyl]hexanediamide; mp 142—143°;

xviii) N-[2-[2-Bromo-3,4-dimethoxyphenyl]ethyl]-N'-[2-phenylethyl]hexanediamide; mp 176—178°;

xix) N-[2-[2-Fluoro-4,5-dimethoxyphenyl]ethyl]-N'-[2-phenylethyl]hexanediamide; mp 142—143°;

xx) N-[2-[4,5-Dimethoxy-2-methylphenyl]ethyl]-N'-[2-phenylethyl]hexanediamide; mp 146—147°;

xxi) N-[2-[3-Fluoro-4,5-dimethoxyphenyl]ethyl]-N'-[2-phenylethyl]hexanediamide; mp 149—150°;

xxii) N-[2-[3-Chloro-4,5-dimethoxyphenyl]ethyl]-N'-[2-phenylethyl]hexanediamide; mp 148.8—150.2°;

xxiii) N-[2-[3-Bromo-4,5-dimethoxyphenyl]ethyl]-N'-[2-phenylethyl]hexanediamide; mp 150.4—152°;

xxiv) N-[2-[3,4-Dimethoxy-5-methylphenyl]ethyl]-N'-[2-phenylethyl]hexanediamide; mp 136.7—137.7°;

xxv) N-[2-[5-Fluoro-3,4-dimethoxy-2-propylphenyl]ethyl]-N'-[2-phenylethyl]hexanediamide; mp 122.6—123.9°;

e) *N-[2-(3,4-Dimethoxy-5-propylphenyl)ethyl]-N'-[2-phenylethyl]-1,6-hexanediamine*

A solution of the bis amide product from step (d) (2.27 g) in dry tetrahydrofuran (100 ml) was stirred under a nitrogen atmosphere while borane in tetrahydrofuran (25 ml of a 1M solution) was added. The solution was heated under reflux for 24 hours.

Methanol (100 ml) was added to the cooled solution and the mixture evaporated to dryness. The residue was dissolved in methanolic HCl (100 ml) and heated under reflux for 2 hours. The solution was evaporated and the sub-title compound as the dihydrochloride salt crystallised from ethanol as colourless prisms (1.9 g) mp 222—224°.

The following compounds were prepared by the method of (e):

i) N-[2-(3,4-Dimethoxy-2-propylphenyl)ethyl]-N'-[2-phenylethyl]-1,6-hexanediamine dihydrochloride; mp 224—225.5°;

ii) N-[2-(3,4-Dimethoxy-2-methylphenyl)ethyl]-N'-[2-phenylethyl]-1,6-hexanediamine dihydrochloride; mp 285—287° dec;

iii) N-[2-(3,4-Dimethoxy-2-nitrophenyl)ethyl]-N'-[2-phenylethyl]-1,6-hexanediamine dihydrochloride; mp 215.6—216.9°;

iv) N-[2-(3,4-Dimethoxy-5-nitrophenyl)ethyl]-N'-[2-phenylethyl]-1,6-hexanediamine dihydrochloride; mp 213—215°;

v) N-[2-(2-Chloro-3,4-dimethoxyphenyl)ethyl]-N'-[2-phenylethyl]-1,6-hexanediamine dihydrochloride; mp 269—272°;

vi) N-[2-[3,4-Dimethoxy-2-[2-phenylethyl]phenyl]ethyl]-N'-[2-phenylethyl]-1,6-hexanediamine dihydrochloride; mp 243—245°;

vii) N-[2-[2-Ethyl-3,4-dimethoxyphenyl]ethyl]-N'-[2-phenylethyl]-1,6-hexanediamine dihydrochloride; mp 249—251°;

viii) N-[2-[2-Butyl-3,4-dimethoxyphenyl]ethyl]-N'-[2-phenylethyl]-1,6-hexanediamine dihydrochloride; mp 209—211°;

ix) N-[2-[3,4-Dimethoxy-2-[1-methylethyl]phenyl]ethyl]-N'-[2-phenylethyl]-1,6-hexanediamine dihydrochloride; mp 233—235°;

x) N-[2-[[1,1'-Biphenyl]-2-yl-5,6-dimethoxy)ethyl]-N'-[2-phenylethyl]-1,6-hexanediamine dihydrochloride; mp 206—208°;

xi) N-[2-[2,3,4-Trimethoxyphenyl]ethyl]-N'-[2-phenylethyl]-1,6-hexanediamine dihydrochloride; mp 243—245°;

xii) N-[2-[3,4-Dimethoxy-2-(phenylmethyl)phenyl]ethyl]-N'-[2-phenylethyl]-1,6-hexanediamine dihydrochloride; mp 194—196°;

xiii) N-[2-[3,4-Dimethoxy-2-(phenylthio)phenyl]ethyl]-N'-[2-phenylethyl]-1,6-hexanediamine dihydrochloride; mp 180.6—182°;

xiv) N-[2-[[1,1'-Biphenyl]-2-yl-4',5,6-trimethoxy]ethyl]-N'-[2-phenylethyl]-1,6-hexanediamine dihydrochloride; mp 236—238°;

xv) N-[2-[2-[2-[4-Methoxyphenyl]ethyl]-3,4-dimethyoxyphenyl]ethyl]-N'-[2-phenylethyl]-1,6-hexanediamine dihydrochloride; mp 221—223°;

xvi) N-[2-[2-Fluoro-3,4-dimethoxyphenyl]ethyl]-N'-[2-phenylethyl]-1,6-hexanediamine dihydrochloride; mpt 275—276° dec;

xvii) N-[2-[2-Bromo-3,4-dimethoxyphenyl]ethyl]-N'-[2-phenylethyl]-1,6-hexanediamine dihydrochloride; mpt 260—262° dec;

xviii) N-[2-[2-Fluoro-4,5-Dimethoxyphenyl]ethyl]-N'-[2-phenylethyl]-1,6-hexanediamine dihydrochloride; mp 258—260° dec;

xvix) N-[2-[4,5-Dimethoxy-2-methylphenyl]ethyl]-N'-[2-phenylethyl]-1,6-hexanediamine dihydrochloride; mp 221—223°;

xx) N-[2-[3-Fluoro-4,5-dimethoxyphenyl]ethyl]-N'-[2-phenylethyl]-1,6-hexanediamine dihydrochloride; mp 260.3—262.3;

xxi) N-[2-[3-Chloro-4,5-dimethoxyphenyl]ethyl]-N'-[2-phenylethyl]-1,6-hexanediamine dihydrochloride; mp 254—256.5 dec;

xxii) N-[2-[3-Bromo-4,5-dimethoxyphenyl]ethyl]-N'-[2-phenylethyl]-1,6-hexanediamine dihydrochloride; mp 251.6—252.9 dec;

xxiii) N-[2-[3,4-Dimethoxy-5-methylphenyl]ethyl]-N'-[2-phenylethyl]-1,6-hexanediamine dihydrochloride; mp 259—262°;

xxiv) N-[2-[5-Fluoro-3,4-dimethoxy-2-propylphenyl]ethyl]-N'-[2-phenylethyl]-1,6-hexanediamine dihydrochloride; mp 225.6—226.8°;

f) *5-[2-(6-(2-Phenylethylamino)hexylamino)ethyl]-3-propyl-1,2-benzenediol dihydrobromide*

A solution of the diamine from step (e) (1.7 g) in 48% aqueous hydrobromic acid (20 ml) containing hypophosphorous acid (0.1 ml) was heated under reflux under a nitrogen atmosphere for 4 hours. The solution was evaporated to dryness and the solid crystallised from 2-propanol/ether to give the hydrobromide of the sub-title compound as colourless prisms (1.3 g) mp 151—152° presoftens 147°.

The following compounds of formula I were prepared by the method of (f):

i) 4-[2-(6-(2-Phenylethylamino)hexylamino)ethyl]-3-propyl-1,2-benzenediol dihydrobromide; mp 95—97°;

ii) 3-Methyl-4-[2-(6-(2-phenylethylamino)hexylamino)ethyl]-1,2-benzenediol dihydrobromide; mp 231—232°;

iii) 3-Nitro-4-[2-(6-(2-phenylethylamino)hexylamino)ethyl]-1,2-benzenediol dihydrobromide; mp 194.6—195.1° dec;

iv) 3-Nitro-5-[2-(6-(2-phenylethylamino)hexylamino)ethyl]-1,2-benzenediol dihydrobromide; mp 245—247° dec;

v) 3-Chloro-4-[2-(6-(2-phenylethylamino)hexylamino)ethyl]-1,2-benzenediol dihydrobromide; mp 209—210°;

vi) 3-[2-Phenylethyl]-4-[2-[6-[2-phenylethylamino]hexylamino]ethyl]-1,2-benzenediol dihydrobromide; mp 143—145°;

vii) 3-Ethyl-4-[2-[6-[2-phenylethylamino]hexylamino]ethyl]-1,2-benzenediol dihydrobromide; mp 178—180°;

viii) 3-Butyl-4-[2-[6-[2-phenylethylamino]hexylamino]ethyl]-1,2-benzenediol dioxalate; mp 198—200°;

ix) 6-[2-[6-[2-Phenylethylamino]hexylamino]ethyl]-1,1'-biphenyl]-2,3-diol dihydrobromide hemihydrate; mp 196—198°;

xi) 4-[2-[6-[2-Phenylethylamino]hexylamino]ethyl]-3-phenylmethyl-1,2-benzenediol dihydrobromide; mp 169—172°;

xii) 3-[2-[4-Hydroxyphenyl]ethyl]-4-[2-[6-[2-phenylethylamino]hexylamino]ethyl]-1,2-benzenediol dihydrobromide; mp 178—180°;

xiii) 3-Fluoro-4-[2-[6-[2-phenylethylamino]hexylamino]ethyl]-1,2-benzenediol dihydrobromide; mp 231—233°;

xiv) 3-Bromo-4-[2-[6-[2-phenylethylamino]hexylamino]ethyl]-1,2-benzenediol dihydrobromide; mp 207—208°;

xv) 5-Fluoro-4-[2-[6-[2-phenylethylamino]hexylamino]ethyl]-1,2-benzenediol dihydrobromide; mp 218—220°;

xvi) 5-Methyl-4-[2-[6-[2-phenylethylamino]hexylamino]ethyl]-1,2-benzenediol dihydrobromide; mp 221—223°;

xvii) 3-Fluoro-5-[2-[6-[2-phenylethylamino]hexylamino]ethyl]-1,2-benzenediol dihydrobromide; mp 207.4—208.4°;

xviii) 3-Methyl-5-[2-[6-[2-phenylethylamino]hexylamino]ethyl]-1,2-benzenediol dihydrobromide; mp 188—191°;

xix) 6-Fluoro-4-[2-[6-[2-phenylethylamino]hexylamino]ethyl]-3-propyl-1,2-benzenediol dihydrobromide; mp 192.6—193.4°.

## Example 2

3-(1-Methylethyl]-4-[2-[6-[2-phenylethylamino]hexylamino]ethyl]-1,2-benzenediol

A suspension of the bis amine prepared by the process of Example 1 (e) (1.7 g) in dry dichloromethane (50 ml) in a nitrogen atmosphere was cooled to −78°. A 1M solution of boron tribromide in dichloromethane (13.6 ml) was added dropwise and the mixture allowed to stir at room temperature for 2 hours. Methanol (50 ml) was slowly added to the mixture and the solution evaporated to dryness and the solid crystallised from ethanol to give the title compound dihydrobromide salt as colourless prisms (1.24 g); mp 221.8—229.8°.

## Example 3

The following compounds of formula I were prepared from the corresponding intermediates of Example 1 (e), by the method of Example 2:

i) 6-[2-[6-[2-Phenylethylamino]hexylamino]ethyl]-[1,1'-biphenyl]-2,3,4'-triol dihydrobromide; mp 239—240°;

ii) 3-Chloro-5-[2-[6-[2-phenylethylamino]hexylamino]ethyl]-1,2-benzenediol dihydrobromide; mp 194.9—197°;

iii) 3-Bromo-5-[2-[6-[2-phenylethylamino]hexylamino]ethyl]-1,2-benzenediol dihydrobromide; mp 184—186°.

Example 4

Benzene methanols analagous to those prepared by the process of Example 1 (a) may also be prepared from the following compounds:

a) *2-[3,4-Dimethoxy-2-(2-phenylethyl)phenyl]-4,5-dihydro-4,4-dimethyloxazole*

A solution of phenylethyl magnesium bromide (1M in tetrahydrofuran (40 ml) was added to a stirred solution of 4,5-dihydro 4,4-dimethyl-2-(2,3,4-trimethoxyphenyl) oxazole (5.3 g 0.02M) in dry tetrahydrofuran under a nitrogen atmosphere. The mixture was stirred at 20° for 16 houurs. Water (200 ml) was added and the aqueous phase thoroughly extracted with diethyl ether (3 × 200 ml). The organic solution was dried over magnesium sulphate, filtered and evaporated to leave an oil which was purified by column chromatography on $SiO_2$ eluting with ether/petroleum ether (50/50)to give a colourless oil (6.2 g); MS m/e 339.

Similarly prepared were:—

i) 2-[2-Ethyl-3,4-dimethoxyphenyl]-4,5-dihydro-4,4-dimethyloxazole; Bp 170—180°/1 mm Hg;

ii) 2-[2-Butyl-3,4-dimethoxyphenyl]-4,5-dihydro-4,4-dimethyloxazole; Bp 170—180°/0.5 mm Hg;

iii) 2-[3,4-Dimethoxy-2-[1-methylethyl]phenyl]-4,5-dihydro-4,4-dimethyloxazole; MS; m/e 277;

iv) 2-[[1,1'-Biphenyl]-2-yl-5,6-dimethoxy]-4,5-dihydro-4,4-dimethyloxazole; mp 107—108.5°;

v) 2-[[1,1'-Biphenyl]-4,5,6-trimethoxy-2-yl]-4,5-dihydro-4,4'-dimethyloxazole; MS; m/e 341;

vi) 4,5-Dihydro-2-[2-[2-[4-methoxyphenyl]ethyl]-3,4-dimethoxyphenyl]-4,4'-dimethyloxazole; MS; m/e 369;

vii) 2-[2-Bromo-3,4-dimethoxyphenyl]-4,5-dihydro-4,4-dimethyloxazole; mp 60—62°.

b) *2-[3,4-Dimethoxy-2-phenylmethylphenyl]-4,5-dihydro-4,4-dimethyloxazole*

A solution of 2-[3,4-Dimethoxyphenyl]-4,5-dihydro-4,4-dimethyloxazole (15 g) in dry tetrahydrofuran (100 ml) under a nitrogen atmosphere was cooled to −45° in a chlorobenzene/dry ice bath. Butyl lithium (45.9 ml 1.6M solution) was added dropwise and the mixture stirred at −45° for 2 hours. Phenylmethyl bromide (38 ml) was then added dropwise and the mixture allowed to stir at 20° for 16 hours. The mixture was quenched with brine and the aqueous phase extracted with ether. The organic phase was separated, dried over magnesium sulphate, filtered and evaporated to leave a solid. Purification by flash chromatography (30% ether/40/60 petrol) gave the title compound as a colourless solid (10.58 g); mp 81—82°.

Similarly prepared were:—

i) 2-[3,4-Dimethoxy-2-[phenylthio]phenyl]-4,5-dihydro-4,4-dimethyloxazole MS; m/e 343.

c) *3,4-Dimethoxy-2-[2-phenylethyl]benzoic acid*

A solution of the product from step (a) (6 g) in excess methyl iodide (11.3 ml) was stirred at 20° for 16 hours and heated under reflux for 2 hours. The solution was evaporated and the solid triturated with ether and dried.

A solution of this oxazolinium salt in 20% aqueous sodium hydroxide (200 ml) and methanol (200 ml) was heated under reflux for 16 hours. The solution was evaporated to half volume, cooled and acidified with 2N HCl. The precipitate was filtered, dried and crystallised from cyclohexane as colourless prisms (3.5 g); mp 142—144°.

Similarly prepared from the corresponding product of step (a) or (b) were:—

i) 2-Butyl-3,4-dimethoxybenzoic acid; mp 112—114°;

ii) 5,6-Dimethoxy-[1,1'-biphenyl]-2-carboxylic acid; mp 198—200°;

iii) 3,4-Dimethoxy-2-phenylmethylbenzoic acid; mp 148—150.5°;

iv) 3,4-Dimethoxy-2-(phenylthio)benzoic acid; mp 168—169.8°

v) 4',5,6-Trimethoxy-[1,1'-biphenyl]-2-carboxylic acid; mp 218—220°;

vi) 2-[2-[4-Methoxyphenyl]ethyl]-3,4-dimethoxybenzoic acid; mp 159—160°;

d) *3,4-Dimethoxy-2-[2-phenylethyl]benzenemethanol*

A 2 Molar solution of borane-tetrahydrofuran complex (12.2 ml) was added to a solution of the product of step (c) (3.5 g) in dry tetrahydrofuran (50 ml) under a nitrogen atmosphere. The solution was heated at reflux for 3 hours, cooled to room temperature, and methanol (50 ml) added. The solution was evaporated to dryness, dissolved in ethyl acetate and washed with dilute hydrochloric acid, saturated sodium bicarbonate solution and water. The organic phase was dried over magnesium sulphate, filtered and evaporated to leave a solid which crystallised from cyclohexane as colourless flakes (3.1 g); mp 99—100°;

Similarly prepared from the appropriate product of step (c) were:—

i) 2-Ethyl-3,4-dmethoxybenzenemethanol; Bp 150/0.5 mm Hg;

ii) 2-Butyl-3,4-dimethoxybenzenemethanol; mp 68—70°;

iii) 5,6-Dimethoxy-[1,1'-biphenyl]-2-methanol; Bp 170/0.5 mm Hg;

iv) 3,4-Dimethoxy-2-phenylmethylbenzenemethanol; Bp 200°/0.15 mm Hg;

v) 3,4-Dimethoxy-2-phenylthiobenzenemethanol; mp 56—58°;
vi) 4',5-6-Trimethoxy-[1,1'-biphenyl]-2-methanol; mp 100—102°;
vii) 2-[2-[4-Methoxyphenyl]ethyl]-3,4-dimethoxybenzenemethanol; mp 83—85°;
viii) 5-Fluoro-3,4-dimethoxy-2-propylbenzenemethanol; MS; m/e 229;

Example 5

Benzaldehydes for the process of Example 1(a) may be prepared by the following route:

a) *3-Acetyloxy-4-methoxy-2-propylbenzaldehyde*

A solution of 3-hydroxy-4-methoxy-2-propylbenzaldehyde (36.6 g) in dry dimethylformamide (50 ml) was added to a suspension of sodium hydride (4.5 g) in dry dimethylformamide (20 ml) in a nitrogen atmosphere. The mixture was stirred at 20° for 30 mins followed by the addition of acetyl chloride (13.5 ml) dropwise over a period of 15 min. The mixture was stirred at 20° for 24 hours.

The solvent was evaporated and the residue treated with dilute hydrochloric acid. The aqueous phase was extracted with ethyl acetate which was washed with brine, dried, filtered and evaporated to leave a yellow oil (39 g); MS; m/e 236.

(b) *3-Acetyloxy-4-methoxy-5-nitro-2-propylbenzaldehyde*

A solution of the product from step (a) (8.8 g) in dry carbon tetrachloride (50 ml) was cooled to −5° during the dropwise addition of fuming nitric acid (19.8 ml) over a period of 1 hour. The mixture was stirred at 0° for 1 hour and then poured onto ice/water. The aqueous phase was extracted with chloroform which was separated, washed with brine, dried, filtered and evaporated to leave a yellow oil (9.9 g); MS m/e 281.

(c) *3-Hydroxy-4-methoxy-5-nitro-2-propylbenzaldehyde*

A suspension of the product from step (b) (39.5 g) in 20% sodium hydroxide solution (140 ml) was heated at 90° for 5 hours. The mixture was cooled and acidified with dilute hydrochloric acid. The aqueous phase was extracted with ethyl acetate washed with brine, dried, filtered and evaporated to leave a brown oil (33 g) MS; m/e 239.

(d) *3,4-Dimethoxy-5-nitro-2-propylbenzaldehyde*

A solution of the product from step (c) (40.2 g), potassium carbonate (46.5 g) and methyl iodide (20.9 ml) in dry dimethylformamide (1560 ml) was stirred at 20°C for 16 hours. The solvent was evaporated and the residue quenched with water. The aqueous phase was extracted with ethyl acetate which was washed with brine, dried, filtered and evaporated to leave a yellow oil (27.4 g), MS; m/e 283.

(e) *2,3-Dimethoxy-5-dimethoxymethyl-4-propylnitrobenzene*

A solution of the aldehyde from step (d) (14 g) tosic acid (0.2 g) and trimethylorthoformate (6.7 ml) in dry methanol (100 ml) was heated under reflux for 30 mins. A solution of potassium hydroxide in methanol (5 ml) was added to the reaction mixture and the solution evaporated to dryness. The residue was dissolved in ether, washed with water, dried, filtered and evaporated to leave a solid which crystallised from pentane as pale yellow prisms (11.6 g); mp 52.1—54.0°.

(f) *3,4-Dimethoxy-5-fluoro-2-propylbenzaldehyde*

The acetal (11.5 g) from step (e) was hydrogenated at atmospheric pressure in dry ethanol (100 ml) using Adam's catalyst. The catalyst was filtered and the solution was evaporated to give the intermediate amine as a green oil (10.5 g) which was used without further purification.

A solution of the amine (5.1 g) in 40% fluoroboric acid (300 ml) was cooled to −5° during the dropwise addition of a cold solution of sodium nitrate (1.45 g) in water (10 ml). The mixture was stirred at 0°C for 1 hour.

The solution was then irradiated with a 400w medium pressure Hg lamp at −10° in a nitrogen atmosphere for 1 hour.

The solution was basified with 40% sodium hydroxide solution and extracted with ethyl acetate. The organic phase was washed with water, dried, filtered and evaporated to leave an oil which was purified by column chromatography ($SiO_2$) eluting with dichloromethane to leave the title aldehyde as a pale yellow oil, MS; m/e 226.

**Claims for the Contracting States: BE CH DE FR GB IT LI LU NL SE**

1. A compound of formula I,

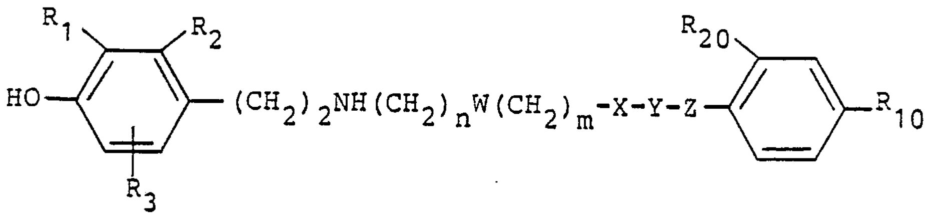

I

in which

$R_1$ represents OH,

$R_2$ and $R_3$, which may be the same or different, each independently represent hydrogen, fluorine, chlorine, bromine, alkyl C1 to 6, nitro, nitrile $(CH_2)_pR_9$ or $SR_9$,

W represents a single bond,

X represents NH,

Y represents $(CH_2)q$, and $R_{20}$ represents hydrogen,

Z represents a single bond,

n and m each independently represent an integer from 1 to 4 inclusive;

q represents an integer from 1 to 3 inclusive;

p represents 0 or an integer from 1 to 3 inclusive;

$R_9$ represents phenyl or phenyl substituted by hydroxy, and

$R_{10}$ represents hydrogen or chlorine,

provided that $R_2$ and $R_3$ do not both represent hydrogen,

and pharmaceutically acceptable salts thereof.

2. A compound according to Claim 1 for use as a pharmaceutical.

3. A compound according to Claim 1, wherein $R_2$ and $R_3$ are selected from hydrogen, fluorine, chlorine, bromine, $CH_2CH_2C_6H_5$ and $CH_2CH_2C_6H_4OH$.

4. 3-Chloro-4-[2-(6-(2-phenylethylamino)hexylamino)ethyl]-1,2-benzenediol;
3-(2-Phenylethyl]-4-[2-[6-[2-phenylethylamino]hexylamino]ethyl]-1,2-benzenediol;
3-Ethyl-4-[2-[6-[2-phenylethylamino]hexylamino]ethyl]-1,2-benzenediol;
3-Bromo-4-[2-[6-[2-phenylethylamino]hexylamino]ethyl]-1,2-benzenediol;
or a pharmaceutically acceptable salt thereof.

5. 5-[2-(6-(2-Phenylethylamino]hexylamino]ethyl]-3-propyl-1,2-benzenediol;
4-[2-(6-(2-Phenylethylamino)hexylamino)ethyl]-3-propyl-1,2-benzenediol;
3-Methyl-4-[2-(6-(2-phenylethylamino)hexylamino)ethyl]-1,2-benzenediol;
3-Nitro-4-[2-(6-(2-phenylethylamino)hexylamino)ethyl]-1,2-benzenediol;
3-Nitro-5-[2-(6-(2-phenylethylamino)hexylamino)ethyl]-1,2-benzenediol;
3-[2-[4-Hydroxyphenyl]ethyl]-4-[2-[6-[2-phenylethylamino)hexylamino]-ethyl]-1,2-benzenediol;
3-Butyl-4-[2-[6-[2-phenylethylamino]hexylamino]ethyl]-1,2-benzenediol;
6-[2-[6-[2-Phenylethylamino]hexylamino]ethyl]-[1,1'-biphenyl]-2,3-diol;
4-[2-[6-[2-Phenylethylamino]hexylamino]ethyl]-3-phenylmethyl-1,2-benzenediol;
3-Fluoro-4-[2-[6-[2-phenylethylamino]hexylamino]ethyl]-1,2-benzenediol;
5-Fluoro-4-[2-[6-[2-phenylethylamino]hexylamino]ethyl]-1,2-benzenediol;
5-Methyl-4-[2-[6-[2-phenylethylamino]hexylamino]ethyl]-1,2-benzenediol;
3-Fluoro-5-[2-[6-[2-phenylethylamino]hexylamino]ethyl]-1,2-benzenediol;
3-Methyl-5-[2-[6-[2-phenylethylamino]hexylamino]ethyl]-1,2-benzenediol;
6-Fluoro-4-[2-[6-[2-phenylethylamino]hexylamino]ethyl]-3-propyl-1,2-benzenediol;
3-[1-Methylethyl]-4-[2-[6-[2-phenylethylamino]hexylamino]ethyl]-1,2-benzenediol;
4-[2-[6-[2-Phenylethylamino]hexylamino]ethyl]-3-phenylthio-1,2-benzenediol;
6-[2-[6-[2-Phenylethylamino]hexylamino]ethyl]-[1,1'-biphenyl]-2,3,4'-triol;
3-Chloro-5-[2-[6-[2-phenylethylamino]hexylamino]ethyl]-1,2-benzenediol;
3-Bromo-5-[2-[6-[2-phenylethylamino]hexylamino]ethyl]-1,2-benzenediol;
or a pharmaceutically acceptable salt thereof.

6. A pharmaceutical composition comprising a compound according to any one of the preceding Claims in admixture with a pharmaceutically acceptable adjuvant, diluent or carrier.

7. A process for the production of a compound of formula I according to Claim 1, or a pharmaceutically acceptable derivative thereof, which comprises removal of at least one protecting group from a compound of formula II,

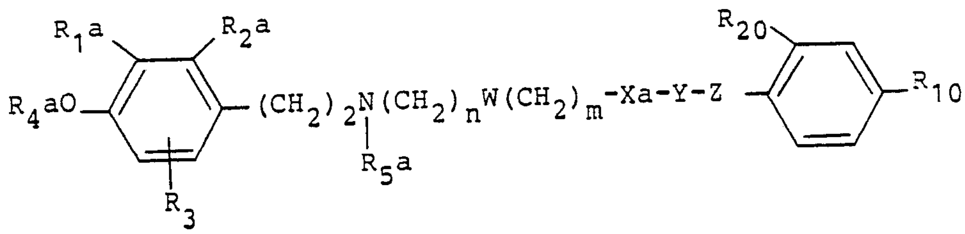
II

in which $R_3$, $R_{10}$, $R_{20}$, n, m, W, Y and Z are as defined in Claim 1,
$R_4a$ and $R_5a$, which may be the same or different, each represent hydrogen or a protecting group,
$R_1a$, $R_2a$ and Xa have the same respective meanings as $R_1$, $R_2$ and X defined in Claim 1, save that in addition
$R_{1a}$ represents $OR_6a$, in which $R_6a$ may represent a protecting group,
Xa may represent $NR_8a$, in which $R_8a$ represents a protecting group,
provided that the compound of formula II bears at least one protecting group,
and where desired or necessary converting the resulting compound of formula I to a pharmaceutically acceptable derivative thereof, or vice versa.

**Claims for the Contracting State: AT**

1. A process for the production of a compound of formula I,

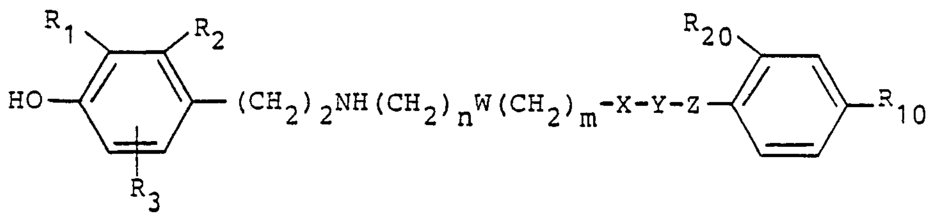
I

in which $R_1$ represents OH,
$R_2$ and $R_3$, which may be the same or different, each independently represent hydrogen, fluorine, chlorine, bromine, alkyl C1 to 6, nitro, nitrile, $(CH_2)_pR_9$ or $SR_9$,
W represents a single bond,
X represents NH,
Y represents $(CH_2)q$, and $R_{20}$ represents hydrogen,
Z represents a single bond,
n and m each independently represent an integer from 1 to 4 inclusive;
q represents an integer from 1 to 3 inclusive;
p represents 0 or an integer from 1 to 3 inclusive;
$R_9$ represents phenyl or phenyl substituted by hydroxy, and
$R_{10}$ represents hydrogen or chlorine,
provided that $R_2$ and $R_3$ do not both represent hydrogen,
and pharmaceutically acceptable salts thereof,
which process comprises removal of at least one protecting group from a compound of formula II,

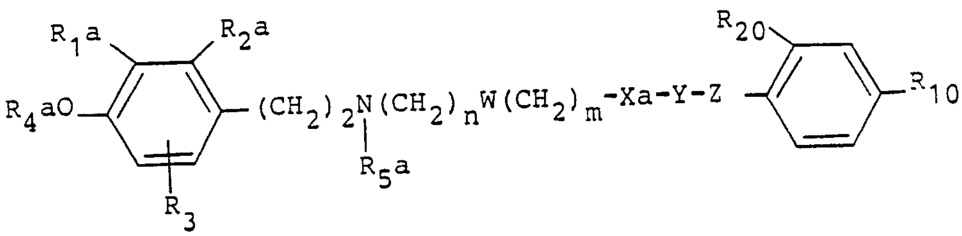
II

in which $R_3$, $R_{10}$, $R_{20}$, n, m, W, Y and Z are as defined above,
$R_4a$ and $R_5a$, which may be the same or different, each represent hydrogen or a protecting group,

$R_1a$, $R_2a$ and Xa have the same respective meanings as $R_1$, $R_2$ and X defined above, save that, in addition,

$R_{1a}$ represents $OR_6a$, in which $R_6a$ may represent a protecting group,

Xa may represent $NR_8a$, in which $R_8a$ represents a protecting group,

provided that the compound of formula II bears at least one protecting group,

and where desired or necessary converting the resulting compound of formula I to a pharmaceutically acceptable derivative thereof, or vice versa.

2. A process according to Claim 1, wherein $R_2$ and $R_3$ are selected from hydrogen, fluorine, chlorine, bromine, $CH_2CH_2C_6H_5$ and $CH_2CH_2C_6H_4OH$.

3. A process according to Claim 1, wherein the compound of formula I is

3-Chloro-4-[2-(6-(2-phenylethylamino)hexylamino)ethyl]-1,2-benzenediol;
3-(2-Phenylethyl]-4-[2-[6-[2-phenylethylamino]hexylamino]ethyl]-1,2-benzenediol;
3-Ethyl-4-[2-[6-[2-phenylethylamino]hexylamino]ethyl]-1,2-benzenediol;
3-Bromo-4-[2-[6-[2-phenylethylamino]hexylamino]ethyl]-1,2-benzenediol;
or a pharmaceutically acceptable salt thereof.

4. A process according to Claim 1, wherein the compound of formula I is

5-[2-(6-(2-Phenylethylamino]hexylamino]ethyl]-3-propyl-1,2-benzenediol;
4-[2-(6-(2-Phenylethylamino)hexylamino)ethyl]-3-propyl-1,2-benzenediol;
3-Methyl-4-[2-(6-(2-phenylethylamino)hexylamino)ethyl]-1,2-benzenediol;
3-Nitro-4-[2-(6-(2-phenylethylamino)hexylamino)ethyl]-1,2-benzenediol;
3-Nitro-5-[2-(6-(2-phenylethylamino)hexylamino)ethyl]-1,2-benzenediol;
3-[2-[4-Hydroxyphenyl]ethyl]-4-[2-[6-[2-phenylethylamino)hexylamino]-ethyl]-1,2-benzenediol;
3-Butyl-4-[2-[6-[2-phenylethylamino]hexylamino]ethyl]-1,2-benzenediol;
6-[2-[6-[2-Phenylethylamino]hexylamino]ethyl]-[1,1′-biphenyl]-2,3-diol;
4-[2-[6-[2-Phenylethylamino]hexylamino]ethyl]-3-phenylmethyl-1,2-benzenediol;
3-Fluoro-4-[2-[6-[2-phenylethylamino]hexylamino]ethyl]-1,2-benzenediol;
5-Fluoro-4-[2-[6-[2-phenylethylamino]hexylamino]ethyl]-1,2-benzenediol;
5-Methyl-4-[2-[6-[2-phenylethylamino]hexylamino]ethyl]-1,2-benzenediol;
3-Fluoro-5-[2-[6-[2-phenylethylamino]hexylamino]ethyl]-1,2-benzenediol;
3-Methyl-5-[2-[6-[2-phenylethylamino]hexylamino]ethyl]-1,2-benzenediol;
6-Fluoro-4-[2-[6-[2-phenylethylamino]hexylamino]ethyl]-3-propyl-1,2-benzenediol;
3-[1-Methylethyl]-4-[2-[6-[2-phenylethylamino]hexylamino]ethyl]-1,2-benzenediol;
4-[2-[6-[2-Phenylethylamino]hexylamino]ethyl]-3-phenylthio-1,2-benzenediol;
6-[2-[6-[2-Phenylethylamino]hexylamino]ethyl]-[1,1′-biphenyl]-2,3,4′-triol;
3-Chloro-5-[2-[6-[2-phenylethylamino]hexylamino]ethyl]-1,2-benzenediol;
3-Bromo-5-[2-[6-[2-phenylethylamino]hexylamino]ethyl]-1,2-benzenediol;
or a pharmaceutically acceptable salt thereof.

**Patentansprüche für die Vertragsstaaten: BE CH DE FR GB IT LI LU NL SE**

1. Verbindung der Formel (I)

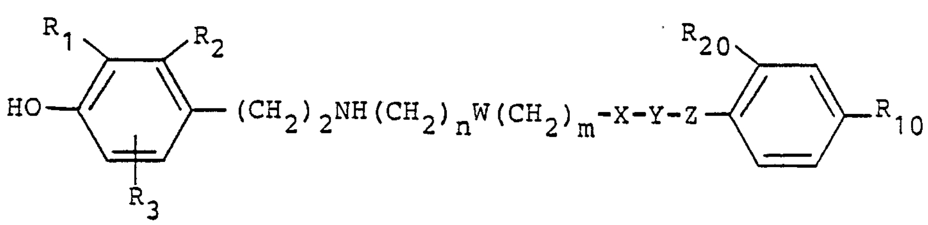

I

worin

$R_1$ OH bedeutet,

$R_2$ und $R_3$, die gleich oder verschieden sein können, jeweils unabhängig Wasserstoff, Fluor, Chlor, Brom, $C_1$—$C_6$-Alkyl, Nitro, Nitril, $(CH_2)_pR_9$ oder $SR_9$ darstellen,

W eine Einfachbindung ist,

X die Bedeutung NH hat,

Y $(CH_2)_q$ bedeutet und $R_{20}$ Wasserstoff ist,

Z eine Einfachbindung bedeutet,

n und m jeweils unabhängig eine ganze Zahl von 1 bis 4 inclusive sind,

q eine ganze Zahl von 1 bis 3 inklusive ist,

p Null oder eine ganze Zahl von 1 bis 3 inklusive ist,

$R_9$ Phenyl oder Phenyl substituiert durch Hydroxy darstellt und

$R_{10}$ Wasserstoff oder Chlor ist,

mit der Maßgabe, daß $R_2$ und $R_3$ nicht beide Wasserstoff bedeuten, und pharmazeutisch annehmbare Salze hievon.

2. Verbindung nach Anspruch 1 zur Verwendung als Pharmazeutikum.

3. Verbindung nach Anspruch 1, worin $R_2$ und $R_3$ ausgewählt sind aus Wasserstoff, Fluor, Chlor, Brom, $CH_2CH_2C_6H_5$ und $CH_2CH_2C_6H_4OH$.

4. 3-Chlor-4-[2-(6-(2-phenyläthylamino)-hexylamino)-äthyl]-1,2-benzoldiol;
3-(2-Phenyläthyl]-4-[2-[6-[2-phenyläthylamino]-hexylamino]-äthyl]-1,2-benzoldiol;
3-Äthyl-4-[2-[6-[2-phenyläthylamino]-hexylamino]-äthyl]-1,2-benzoldiol;
3-Brom-4-[2-[6-[2-phenyläthylamino]-hexylamino]-äthyl]-1,2-benzoldiol;
oder ein pharmazeutisch annehmbares Salz hievon.

5. 5-[2-(6-(2-Phenyläthylamino]-hexylamino]-äthyl]-3-propyl-1,2-benzoldiol;
4-[2-(6-(2-Phenyläthylamino)-hexylamino)-äthyl]-3-propyl-1,2-benzoldiol;
3-Methyl-4-[2-(6-(2-phenyläthylamino)-hexylamino)-äthyl]-1,2-benzoldiol;
3-Nitro-4-[2-(6-(2-phenyläthylamino)-hexylamino)-äthyl]-1,2-benzoldiol;
3-Nitro-5-[2-(6-(2-phenyläthylamino)-hexylamino)-äthyl]-1,2-benzoldiol;
3-[2-[4-Hydroxyphenyl]-äthyl]-4-[2-[6-[2-phenyläthylamino)-hexylamino]-äthyl]-1,2-benzoldiol;
3-Butyl-4-[2-[6-[2-phenyläthylamino]-hexylamino]-äthyl]-1,2-benzoldiol;
6-[2-[6-[2-Phenyläthylamino]-hexylamino]-äthyl]-[1,1'-biphenyl]-2,3-diol;
4-[2-[6-[2-Phenyläthylamino]-hexylamino]-äthyl]-3-phenylmethyl-1,2-benzoldiol;
3-Fluor-4-[2-[6-[2-phenyläthylamino]-hexylamino]-äthyl]-1,2-benzoldiol;
5-Fluor-4-[2-[6-[2-phenyläthylamino]-hexylamino]-äthyl]-1,2-benzoldiol;
5-Methyl-4-[2-[6-[2-phenyläthylamino]-hexylamino]-äthyl]-1,2-benzoldiol;
3-Fluor-5-[2-[6-[2-phenyläthylamino]-hexylamino]-äthyl]-1,2-benzoldiol;
3-Methyl-5-[2-[6-[2-phenyläthylamino]-hexylamino]-äthyl]-1,2-benzoldiol;
6-Fluor-4-[2-[6-[2-phenyläthylamino]-hexylamino]-äthyl]-3-propyl-1,2-benzoldiol;
3-[1-Methyläthyl]-4-[2-[6-[2-phenyläthylamino]-hexylamino]-äthyl]-1,2-benzoldiol;
4-[2-[6-[2-Phenyläthylamino]-hexylamino]-äthyl]-3-phenylthio-1,2-benzoldiol;
6-[2-[6-[2-Phenyläthylamino]-hexylamino]-äthyl]-[1,1'-biphenyl]-2,3,4'-triol;
3-Chloro-5-[2-[6-[2-phenyläthylamino]-hexylamino]-äthyl]-1,2-benzoldiol;
3-Brom-5-[2-[6-[2-phenyläthylamino]-hexylamino]-äthyl]-1,2-benzoldiol;
oder ein pharmazeutisch annehmbares Salz hievon.

6. Pharmazeutische Zusammensetzung umfassend eine Verbindung nach einem der vorhergehenden Ansprüche in Mischung mit einem pharmazeutisch annehmbaren Adjuvans, Verdünnungsmittel oder Träger.

7. Verfahren zur Herstellung einer Verbindung der Formel (I) nach Anspruch 1 oder eines pharmazeutisch annehmbaren Derivates hievon, das die Entfernung mindestens einer Schutzgruppe von einer Verbindung der Formel (II)

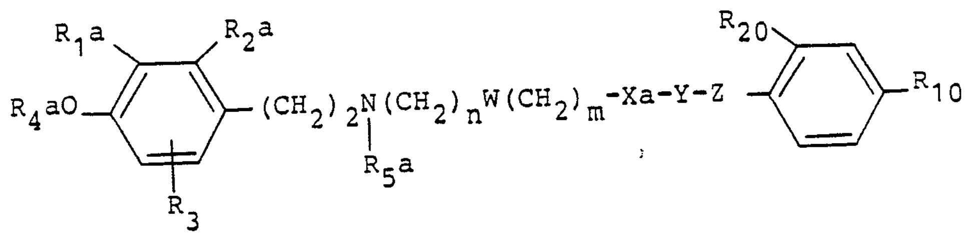

II

worin $R_3$, $R_{10}$, $R_{20}$, n, m, W, Y und Z wie in Anspruch 1 definiert sind, $R_4a$ und $R_5a$, die gleich oder verschieden sein können, jeweils Wasserstoff oder eine Schutzgruppe bedeuten, $R_1a$, $R_2a$ und Xa die gleichen Bedeutungen wie $R_1$, $R_2$ und X haben, wie in Anspruch 1 definiert, außer daß zusätzlich

$R_1a$ die Bedeutung $OR_6a$ hat, wobei $R_6a$ eine Schutzgruppe bedeuten kann,

Xa $NR_8a$ sein kann, wobei $R_8a$ eine Schutzgruppe bedeutet,

mit der Maßgabe, daß die Verbindung der Formel (II) mindestens eine Schutzgruppe trägt, und, wenn gewünscht oder notwendig, das Überführen der erhaltenen Verbindung der Formel (I) in ein pharmazeutisch annehmbares Derivat hievon oder vice versa umfaßt.

**Patentansprüche für den Vertragsstaat: AT**

1. Verfahren zur Herstellung einer Verbindung der Formel (I)

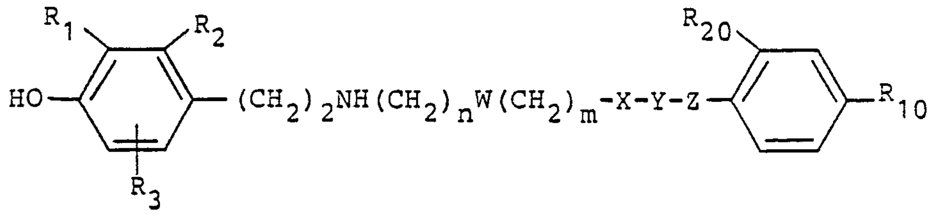

I

worin

$R_1$ OH bedeutet,

$R_2$ und $R_3$, die gleich oder verschieden sein können, jeweils unabhängig Wasserstoff, Fluor, Chlor, Brom, $C_1$—$C_6$-Alkyl, Nitro, Nitril, $(CH_2)_pR_9$ oder $SR_9$ darstellen,

W eine Einfachbindung ist,

X die Bedeutung NH hat,

Y $(CH_2)_q$ bedeutet und $R_{20}$ Wasserstoff ist,

Z eine Einfachbindung bedeutet,

n und m jeweils unabhängig eine ganze Zahl von 1 bis 4 inklusive sind,

q eine ganze Zahl von 1 bis 3 inklusive ist,

p Null oder eine ganze Zahl von 1 bis 3 inklusive ist,

$R_9$ Phenyl oder Phenyl substituiert durch Hydroxy darstellt und

$R_{10}$ Wasserstoff oder Chlor ist,

mit der Maßgabe, daß $R_2$ und $R_3$ nicht beide Wasserstoff bedeuten, und pharmazeutisch annehmbarer Salze hievon, welches Verfahren die Entfernung mindestens einer Schutzgruppe von einer Verbindung der Formel (II)

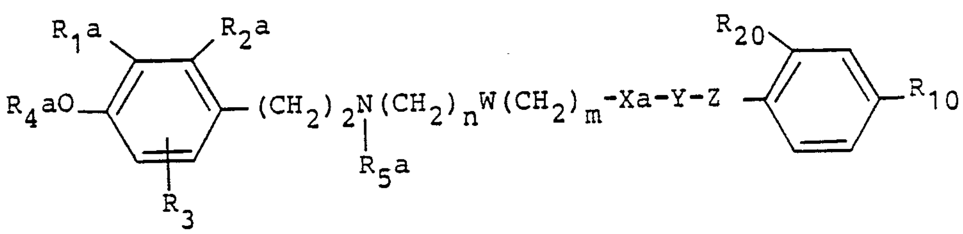

II

worin $R_3$, $R_{10}$, $R_{20}$, n, m, W, Y und Z wie oben definiert sind, $R_4a$ und $R_5a$, die gleich oder verschieden sein können, jeweils Wasserstoff oder eine Schutzgruppe bedeuten, $R_1a$, $R_2a$ und Xa die gleichen Bedeutungen wie $R_1$, $R_2$ und X haben, wie oben definiert, außer daß zusätzlich

$R_1a$ die Bedeutung $OR_6a$ hat, wobei $R_6a$ eine Schutzgruppe bedeuten kann,

Xa $NR_8a$ sein kann, wobei $R_8a$ eine Schutzgruppe bedeutet,

mit der Maßgabe, daß die Verbindung der Formel (II) mindestens eine Schutzgruppe trägt, und, wenn gewünscht oder notwendig, das Überführen der erhaltenen Verbindung der Formel (I) in ein pharmazeutisch annehmbares Derivat hievon oder vice versa umfaßt.

2. Verfahren nach Anspruch 1, worin $R_2$ und $R_3$ ausgewählt sind aus Wasserstoff, Fluor, Chlor, Brom, $CH_2CH_2C_6H_5$ und $CH_2CH_2C_6H_4OH$.

3. Verfahren nach Anspruch 1, worin die Verbindung der Formel (I) 3-Chlor-4-[2-(6-(2-phenyläthylamino)-hexylamino)-äthyl]-1,2-benzoldiol;

3-(2-Phenyläthyl)-4-[2-[6-[2-phenyläthylamino]-hexylamino]-äthyl]-1,2-benzoldiol;

3-Äthyl-4-[2-[6-[2-phenyläthylamino]-hexylamino]-äthyl]-1,2-benzoldiol;

3-Brom-4-[2-[6-[2-phenyläthylamino]-hexylamino]-äthyl]-1,2-benzoldiol;

oder ein pharmazeutisch annehmbares Salz hievon ist.

4. Verfahren nach Anspruch 1, worin die Verbindung der Formel (I) 5-[2-(6-(2-Phenyläthylamino)-hexylamino)-äthyl]-3-propyl-1,2-benzoldiol;

4-[2-(6-(2-Phenyläthylamino)-hexylamino)-äthyl]-3-propyl-1,2-benzoldiol;

3-Methyl-4-[2-(6-(2-phenyläthylamino)-hexylamino)-äthyl]-1,2-benzoldiol;

3-Nitro-4-[2-(6-(2-phenyläthylamino)-hexylamino)-äthyl]-1,2-benzoldiol;

3-Nitro-5-[2-(6-(2-phenyläthylamino)-hexylamino)-äthyl]-1,2-benzoldiol;

3-[2-[4-Hydroxyphenyl]-äthyl]-4-[2-[6-[2-phenyläthylamino)-hexylamino]-äthyl]-1,2-benzoldiol;

3-Butyl-4-[2-[6-[2-phenyläthylamino]-hexylamino]-äthyl]-1,2-benzoldiol;

6-[2-[6-[2-Phenyläthylamino]-hexylamino]-äthyl]-[1,1'-biphenyl]-2,3-diol;

4-[2-[6-[2-Phenyläthylamino]-hexylamino]-äthyl]-3-phenylmethyl-1,2-benzoldiol;
3-Fluor-4-[2-[6-[2-phenyläthylamino]-hexylamino]-äthyl]-1,2-benzoldiol;
5-Fluoro-4-[2-[6-[2-phenyläthylamino]-hexylamino]-äthyl]-1,2-benzoldiol;
5-Methyl-4-[2-[6-[2-phenyläthylamino]-hexylamino]-äthyl]-1,2-benzoldiol;
3-Fluor-5-[2-[6-[2-phenyläthylamino]-hexylamino]-äthyl]-1,2-benzoldiol;
3-Methyl-5-[2-[6-[2-phenyläthylamino]-hexylamino]-äthyl]-1,2-benzoldiol;
6-Fluor-4-[2-[6-[2-phenyläthylamino]-hexylamino]-äthyl]-3-propyl-1,2-benzoldiol;
3-[1-Methyläthyl]-4-[2-[6-[2-phenyläthylamino]-hexylamino]-äthyl]-1,2-benzoldiol;
4-[2-[6-[2-Phenyläthylamino]-hexylamino]-äthyl]-3-phenylthio-1,2-benzoldiol;
6-[2-[6-[2-Phenyläthylamino]-hexylamino]-äthyl]-[1,1'-biphenyl]-2,3,4'-triol;
3-Chloro-5-[2-[6-[2-phenyläthylamino]-hexylamino]-äthyl]-1,2-benzoldiol;
3-Brom-5-[2-[6-[2-phenyläthylamino]-hexylamino]-äthyl]-1,2-benzoldiol

oder ein pharmazeutisch annehmbares Salz hievon ist.

**Revendications pour les Etats contractants: BE CH DE FR GB IT LI LU NL SE**

1. Composéde formule I,

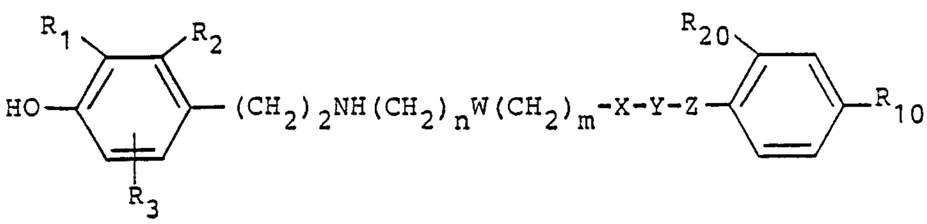

I

où

$R_1$ représente OH,
$R_2$ et $R_3$, qui peuvent être identiques ou différents, représentent chacun hydrogène, fluor, chlore, brome, alcoyle en C1 à 6, nitro, $(CH_2)_pR_9$ ou $SR_9$,
W représente une liaison simple,
X représente NH,
Y représente $(CH_2)_q$,
$R_{20}$ représente hydrogène,
Z représente une liaison simple,
n et m représentent chacun indépendamment un entier de 1 à 4 inclusivement,
q représente un entier de 1 à 3 inclusivement,
p représente 0 ou un entier de 1 à 3 inclusivement,
$R_9$ représente phényle ou phényle substitué par hydroxyle, et
$R_{10}$ représente hydrogène ou chlore,
avec la restriction que $R_2$ et $R_3$ ne représentent pas tous deux hydrogène,

et les sels pharmaceutiquement acceptables de celui-ci.

2. Composé suivant la revendication 1 à utiliser comme agent pharmaceutique.

3. Composé suivant la revendication 1, dans lequel $R_2$ et $R_3$ sont choisis parmi hydrogène, fluor, chlore, brome, $CH_2CH_2C_6H_5$ et $CH_2CH_2C_6H_4OH$.

4. Le 3-chloro-4-[2-(6-(2-phényléthylamino)hexylamino)éthyl]-1,2-benzènediol;
le 3-[2-phényléthyl]-4-[2-[6-[2-phényléthylamino]hexylamino]éthyl]-1,2-benzènediol;
le 3-éthyl-4-[2-[6-[2-phényléthylamino]hexylamino]éthyl]-1,2-benzènediol;
le 3-bromo-4-[2-[6-[2-phényléthylamino]hexylamino]éthyl]-1,2-benzènediol;
ou un sel pharmaceutiquement acceptable de l'un de ceux-ci.

5. Le 5-[2-(6-(2-phényléthylamino)-hexylamino)éthyl]-3-propyl-1,2-benzènediol;
le 4-[2-(6-(2-phényléthylamino)-hexylamino)éthyl]-3-propyl-1,2-benzènediol;
le 3-méthyl-4-[2-(6-(2-phényléthylamino)hexylamino)-éthyl]-1,2-benzènediol;
le 3-nitro-4-[2-(6-(2-phényléthylamino)-hexylamino)-éthyl]-1,2-benzènediol;
le 3-nitro-5-[2-(6-(2-phényléthylamino)hexylamino)-éthyl]-1,2-benzènediol;
le 3-[2-[4-hydroxyphényl]éthyl]-4-[2-[6-2-phényléthylamino)-hexylamino]éthyl]-1,2-benzènediol;
le 3-butyl-4-[2-[6-[2-phényléthylamino]-hexylamino]éthyl]-1,2-benzènediol;
le 6-[2-[6-[2-phényléthylamino]hexylamino]éthyl]-[1,1'-biphényl]-2,3-diol;
le 4-[2-[6-[2-phényléthylamino]hexylamino]éthyl]-3-phénylméthyl-1,2-benzènediol;
le 3-fluoro-4-[2-[6-[2-phényléthylamino]hexylamino]éthyl]-1,2-benzènediol;
le 5-fluoro-4-[2-[6-[2-phényléthylamino]hexylamino]éthyl]-1,2-benzènediol;
le 5-méthyl-4-[2-[6-[2-phényléthylamino]hexylamino]éthyl]-1,2-benzènediol;
le 3-fluoro-5-[2-[6-[2-phényléthylamino]hexylamino]éthyl]-1,2-benzènediol;
le 3-méthyl-5-[2-[6-[2-phényléthylamino]hexylamino]éthyl]-1,2-benzènediol;

le 6-fluoro-4-[2-[6-[2-phényléthylamino]hexylamino]éthyl]-3-propyl-1,2-benzènediol;
le 3-[1-méthyléthyl]-4-[2-[6-[2-phényléthylamino]hexylamino]éthyl]-1,2-benzènediol;
le 4-[2-[6-[2-phényléthylamino]hexylamino]éthyl]-3-phénylthio-1,2-benzènediol;
le 6-[2-[6-[2-phényléthylamino]hexylamino]éthyl]-[1,1'-biphényl]-2,3,4'-triol;
le 3-chloro-5-[2-[6-[2-phényléthylamino]hexylamino]éthyl]-1,2-benzènediol;
le 3-bromo-5-[2-[6-[2-phényléthylamino]hexylamino]éthyl]-1,2-benzènediol;
ou un sel pharmaceutiquement acceptable de l'un de ceux-ci.

6. Composition pharmaceutique, comprenant un composé suivant l'une quelconque des revendications précédentes en mélange avec un adjuvant, diluant ou excipient pharmaceutiquement acceptable.

7. Procédé de préparation d'un composé de formule I suivant la revendication 1, ou d'un dérivé pharmaceutiquement acceptable de celui-ci, qui comprend l'élimination d'au moins un radical protecteur d'un composé de formule II,

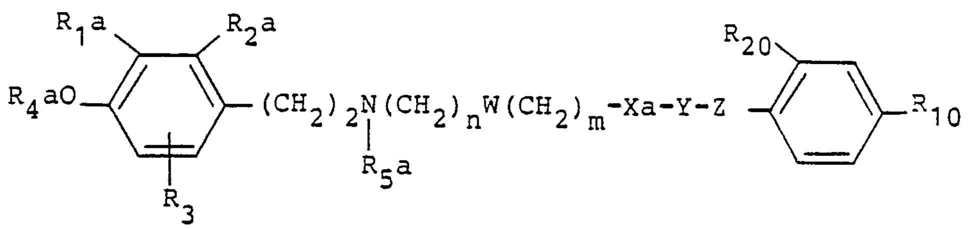

II

où $R_3$, $R_{10}$, $R_{20}$, n, m, W, Y et Z sont tels que définis dans la revendication 1,

$R_4a$ et $R_5a$, qui peuvent être identiques ou différents, représentent chacun hydrogène ou un radical protecteur,

$R_1a$, $R_2a$ et Xa ont les mêmes significations respectives que $R_1$, $R_2$ et X définis dans la revendication 1, sauf qu'en outre

$R_1a$ représente $OR_6a$, où $R_6a$ peut représenter un radical protecteur,

Xa peut représenter $NR_8a$, où $R_8a$ représente un radical protecteur,

avec la restriction que le composé de formule II porte au moins un radical protecteur,

et lorsque la chose est souhaitée ou nécessaire, la conversion du composé résultant de formule I en un sel pharmaceutiquement acceptable de celui-ci, et vice versa.

**Revendications pour l'Etat contractant: AT**

1. Procédé de préparation d'un composé de formule I,

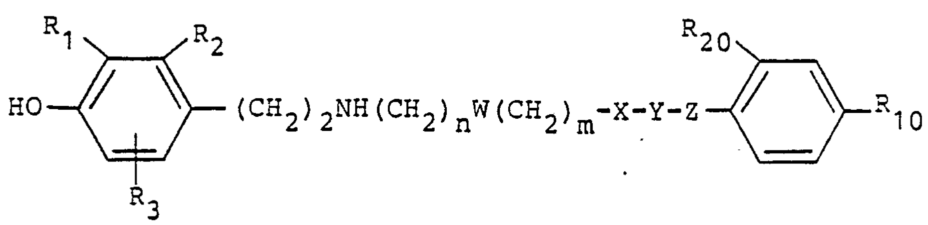

I

où

$R_1$ représente OH,

$R_2$ et $R_3$, qui peuvent être identiques ou différents, représentent chacun hydrogène, fluor, chlore, brome, alcoyle en C1 à 6, nitro, $(CH_2)_pR_9$ ou $SR_9$,

W représente une liaison simple,

X représente NH,

Y représente $(CH_2)_q$,

$R_{20}$ représente hydrogène,

Z représente une liaison simple,

n et m représentent chacun indépendamment un entier de 1 à 4 inclusivement,

q représentente un entier de 1 à 3 inclusivement,

p représente 0 ou un entier de 1 à 3 inclusivement,

$R_9$ représente phényle ou phényle substitué par hydroxyle, et

$R_{10}$ représente hydrogène ou chlore,

avec la restriction que $R_2$ et $R_3$ ne représentent pas tous deux hydrogène,

et des sels pharmaceutiquement acceptables de celui-ci; lequel procédé comprend l'élimination d'au moins un radical protecteur d'un composé de formule II,

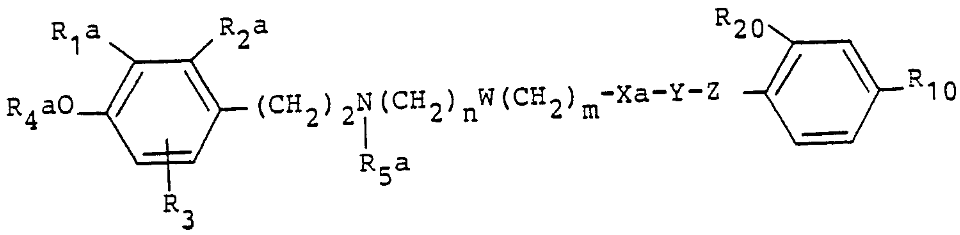

II

où $R_3$, $R_{10}$, $R_{20}$, n, m, W, Y et Z sont tels que définis ci-dessus,

$R_4a$ et $R_5a$, qui peuvent être identiques ou différents, représentent chacun hydrogène ou un radical protecteur,

$R_1a$, $R_2a$ et Xa ont les mêmes significations respectives que $R_1$, $R_2$ et X définis ci-dessus, sauf qu'en outre

$R_1a$ représente $OR_6a$, où $R_6a$ peut représenter un radical protecteur,

Xa peut représenter $NR_8a$, où $R_8a$ représente un radical protecteur,

avec la restriction que le composé de formule II porte au moins un radical protecteur,

et lorsque la chose est souhaitée ou nécessaire, la conversion du composé résultant de formule I en un sel pharmaceutiquement acceptable de celui-ci, et vice versa.

2. Procédé suivant la revendication 1, dans lequel $R_2$ et $R_3$ sont choisis parmi hydrogène, fluor, chlore, brome, $CH_2CH_2C_6H_5$ et $CH_2CH_2C_6H_4OH$.

3. Procédé suivant la revendication 1, dans lequel le composé de formule I est
le 3-chloro-4-[2-(6-(2-phényléthylamino)hexylamino)éthyl]-1,2-benzènediol;
le 3-(2-phényléthyl]-4-[2-[6-[2-phényléthylamino]hexylamino]éthyl]-1,2-benzènediol;
le 3-éthyl-4-[2-[6-[2-phényléthylamino]hexylamino]éthyl]-1,2-benzènediol;
le 3-bromo-4-[2-[6-[2-phényléthylamino]hexylamino]éthyl]-1,2-benzènediol;
ou un sel pharmaceutiquement acceptable de l'un de ceux-ci.

4. Procédé suivant la revendication 1, dans lequel le composé de formule I est
le 5-[2-(6-(2-phényléthylamino)-hexylamino)éthyl]-3-propyl-1,2-benzènediol;
le 4-[2-(6-(2-phényléthylamino)-hexylamino)éthyl]-3-propyl-1,2-benzènediol;
le 3-méthyl-4-[2-(6-(2-phényléthylamino)hexylamino)-éthyl]-1,2-benzènediol;
le 3-nitro-4-[2-(6-(2-phényléthylamino)-hexylamino)-éthyl]-1,2-benzènediol;
le 3-nitro-5-[2-(6-(2-phényléthylamino)hexylamino)-éthyl]-1,2-benzènediol;
le 3-[2-[4-hydroxyphényl]éthyl]-4-[2-[6-(2-phénylèthylamino)-hexylamino]éthyl]-1,2-benzènediol;
le 3-butyl-4-[2-[6-[2-phényléthylamino]-hexylamino]éthyl]-1,2-benzènediol;
le 6-[2-[6-[2-phényléthylamino]hexylamino]éthyl]-[1,1'-biphényl]-2,3-diol;
le 4-[2-[6-[2-phényléthylamino]hexylamino]éthyl]-3-phénylméthyl-1,2-benzènediol;
le 3-fluoro-4-[2-[6-[2-phényléthylamino]hexylamino]éthyl]-1,2-benzènediol;
le 5-fluoro-4-[2-[6-[2-phényléthylamino]hexylamino]éthyl]-1,2-benzènediol;
le 5-méthyl-4-[2-[6-[2-phényléthylamino]hexylamino]éthyl]-1,2-benzènediol;
le 3-fluoro-5-[2-[6-[2-phényléthylamino]hexylamino]éthyl]-1,2-benzènediol;
le 3-méthyl-5-[2-[6-[2-phényléthylamino]hexylamino]éthyl]-1,2-benzènediol;
le 6-fluoro-4-[2-[6-[2-phényléthylamino]hexylamino]éthyl]-3-propyl-1,2-benzènediol;
le 3-[1-méthyléthyl]-4-[2-[6-[2-phényléthylamino]hexylamino]éthyl]-1,2-benzènediol;
le 4-[2-[6-[2-phényléthylamino]hexylamino]éthyl]-3-phénylthio-1,2-benzènediol;
le 6-[2-[6-[2-phényléthylamino]hexylamino]éthyl]-[1,1'-biphényl]-2,3,4'-triol;
le 3-chloro-5-[2-[6-[2-phényléthylamino]hexylamino]éthyl]-1,2-benzènediol;
le 3-bromo-5-[2-[6-[2-phényléthylamino]hexylamino]éthyl]-1,2-benzènediol;
ou un sel pharmaceutiquement acceptable de l'un de ceux-ci.